# EUROPEAN PATENT SPECIFICATION

(11) **EP 1 471 062 B1**
(45) Date of publication and mention of the grant of the patent: **03.06.2009**
(21) Application number: 02792008.1
(22) Date of filing: 26.12.2002
(51) Int. Cl.: C07D 493/20

(54) **FULLERENE EPOXIDE AND PROCESS FOR PRODUCING THE SAME**
FULLERENEPOXID UND VERFAHREN ZU DESSEN HERSTELLUNG
FULLERENE EPOXYDIQUE ET PROCEDE DE PRODUCTION DE CE DERNIER

(30) Priority: 27.12.2001 JP 2001397312
(43) Date of publication of application: 27.10.2004
(73) Proprietor: RIKEN, Wako-shi, Saitama 351-0198 (JP)
(72) Inventor: TAJIMA, Yusuke, c/o RIKEN, Wako-shi, Saitama 351-0198 (JP); TAKEUCHI, Kazuo, c/o RIKEN, Wako-shi, Saitama 351-0198 (JP)
(74) Representative: Gervasi, Gemma
(86) International application number: PCT/JP2002/013626
(87) International publication number: WO 2003/055870

(56) References cited:
- JP-A- 6 321 517
- HAMANO T. ET AL: "Oxidation of [60]Fullerene by Cytochrome P450 Chemical Models" JOURNAL OF THE CHEMICAL SOCIETY, CHEMICAL COMMUNICATIONS, no. 15, 1995, pages 1537-1538, XP002347854 London, U.K.
- TAJIMA Y., TAKEUCHI K.: 'Discovery of C6003Isomer having C3vSymmetry' J. ORG. CHEM. vol. 67, no. 5, 2002, pages 1696 - 1698, XP002967019
- FUSCO C. ET AL.: 'Oxyfunctionalization of non-natural targets by dioxiranes' J. ORG. CHEM. vol. 64, no. 22, 1999, pages 8363 - 8368, XP002967020

## Description

### Technical Field

The present invention relates to fullerene epoxide, a method of manufacturing the same, and a method of separating the same.

### Background Art

Fullerene epoxide has attracted attention in the fields of cluster science relating to clathrate compounds and application to pharmaceuticals and photo-electronic devices. Fullerene epoxide has also garnered attention in the areas of application to the manufacturing of giant carbon clusters and fullerene polymers.

Fullerene epoxide is produced by oxidizing fullerene under prescribed conditions. Some isomers of fullerene C₆₀ epoxide having three epoxide groups per fullerene molecule have been confirmed, and the existence of others has been predicted (Beck, R.D. et al., J. Chem. Phys. 1994,101(4), 3243-3429, Fusco, C. et al., J. Org. Chem. 1999, 64, 8363-8368, and Hamano T. et al., J-Chem.Soc-, Chem-Commun-, 1995, 15, 1537-1538)

There are also predicted isomers for which no manufacturing method applicable to fullerene C₆₀ epoxide having three epoxide groups per fullerene molecule, and in particular, no isomer isolation method, have been established. Nor has the usefulness thereof been confirmed.

Accordingly, the present invention has for its object to provide new fullerene C₆₀ epoxides having three epoxide groups. In particular, the present invention has for its object to provide a new fullerene C₆₀ epoxide having three epoxide groups and having C₁, symmetry.

The present invention has a further object of providing a method of separating fullerene epoxides into isomers and a method of manufacturing fullerene C₆₀ epoxides having three epoxide groups using this separation method.

### Summary of the Invention

(1) A fullerene epoxide denoted as C₆₀O₃, characterized by the presence of two epoxide groups in a single six-membered ring, and by the presence of one epoxide group in a six-membered ring adjacent to the six-membered ring in which said two epoxide groups are present or by the presence of one epoxide group in a six-membered ring one six-membered ring removed from the six-membered ring in which said two epoxide groups are present.
(2) The fullerene epoxide according to (1), denoted as C₆₀O₃, having three epoxide groups and having C₁, symmetry.
(3) The fullerene epoxide according to (1) or (2) having C₁ symmetry.
(4) The fullerene epoxide according to (3) in the form of a racemic or an enantiomer.
(5) A method of manufacturing fullerene epoxide comprising:
   separating a fraction containing only fullerene triepoxides from a mixture of fullerene epoxides using silica gel, and
   separating the fraction containing only fullerene triepoxides thus obtained using a substance having π-π interaction to obtain the fullerene epoxide according to any of (1) to (4).
(6) The method of manufacturing according to (5) wherein said silica gel is alkane group-bonded silica gel.
(7) A method of separating fullerene epoxide characterized in that a fullerene epoxide mixture is separated using a substance having π-π interaction.
(8) The method according to any of (5) to (7) wherein the substance having π-π interaction is pyrene-modified silica gel.

### Brief Description of the Figures

Fig. 1 shows the structure, dipole moment µ (calculated value), and heat of formation H.F. (calculated value) of fullerene epoxide C₆₀O₃ having Cₛ symmetry (3a), fullerene epoxide C₆₀O₃ having C₂ symmetry (3b), and fullerene epoxide C₆₀O₃ having C₁ symmetry (3c).
Fig. 2 is an HPLC chromatogram of a fullerene epoxide mixture.
Fig. 3 shows a chromatogram obtained by eluting peak f with toluene/methanol (60:40) on a Develosil C30 RPFULLERENE column (top), and a chromatogram obtained by eluting peak f with toluene/methanol (80:20) on a Cosmosil Bucky Prep column (bottom).
Fig. 4 shows visible absorption spectra of the peak e fraction and the peak I and II fractions.
Fig. 5 is the result of ¹³C-NMR measurement of isomers obtained from the peak II fraction identifying three fullerene C₆₀ epoxides (3a) having Cₛ symmetry.
Fig. 6 is the result of ¹³C-NMR measurement of isomers obtained from the peak II fraction identifying three fullerene C₆₀ epoxides (3a) having Cₛ symmetry.
Fig. 7 is the result of ¹³C-NMR measurement of isomers obtained from the peak I fraction identifying three fullerene C₆₀ epoxides (3b) having C₂ symmetry.
Fig. 8 is the result of ¹³C-NMR measurement of isomers obtained from the peak I fraction identifying three fullerene C₆₀ epoxides (3b) having C₂ symmetry.
Fig. 9 is the result of ¹³C-NMR measurement of isomers obtained from the peak e fraction identifying three fullerene C₆₀ epoxides (3c) having C₁ symmetry.
Fig. 10 is the result of ¹³C-NMR measurement of isomers obtained from the peak e fraction identifying three fullerene C₆₀ epoxides (3c) having C₁ symmetry.
Fig. 11 is the result of measurement of the first reduction potential (E1) of each of the isomers of C₆₀O₃ having C₁, C₂, and Cₛ symmetry, and the lowest unoccupied molecular orbit (LUMO) level obtained by semiempirical molecular orbit calculation.
Fig. 12 shows the FT-IR spectra of the fractions of peaks I, II and e, and C₆₀, C₆₀O, and C₆₀O₂.
Fig. 13 is a CD spectrum of C₆₀O₃ isomer (3c).

### Best Mode of Implementing the Invention

The fullerene epoxide of the present invention is denoted as C₆₀O₃ and is characterized by the presence of two epoxide groups in a single six-membered ring, and by the presence of one epoxide group in a six-membered ring adjacent to the six-membered ring in which said two epoxide groups are present, or by the presence of one epoxide group in a six-membered ring one six-membered ring removed from the six-membered ring in which said two epoxide groups are present.

Based on the number of epoxide groups present in fullerene C₆₀, there exist numerous fullerene C₆₀ epoxides. Of these, the present invention covers the compounds, denoted as C₆₀O₃, in which there are three epoxide groups in fullerene C₆₀.

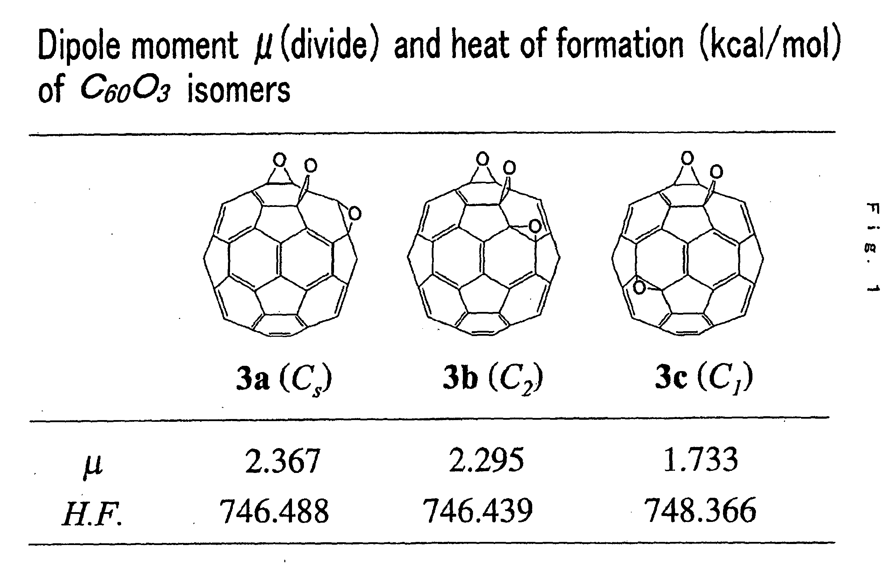

Fullerene epoxides denoted as C₆₀O₃ in which two epoxide groups are present on a single six-membered ring and in which one epoxide is present in a six-membered ring adjacent to the six-membered ring on which the two epoxide groups are present are, for example, compounds having C₂ or Cₛ symmetry. Fullerene epoxides having C₂ symmetry have the structure denoted in Fig. 1 as 3b, and fullerene epoxides having Cₛ symmetry have the structure denoted in Fig. 1 as 3a.

Fullerene epoxides denoted as C₆₀O₃ in which two epoxide groups are present on a single six-membered ring and in which one epoxide is present on a six-membered ring one six-membered ring removed from the six-membered ring on which the two epoxide groups are present are, for example, compounds having C₁ symmetry. Fullerene epoxides having C₁ symmetry have the structure denoted in Fig. 1 as 3c.

The existence of fullerene C₆₀ epoxide (isomer) having Cₛ symmetry and fullerene C₆₀ epoxide (isomer) having C₂ symmetry has been predicted (Non-patent Reference 2 (Fusco, C. et al., J. Org. Chem. 1999, 64, 8363-8368)). However, they have never before been isolated and identified as substances.

Further, no fullerene C₆₀ epoxide (isomer) having C₁ symmetry has ever been reported

The present invention permits the manufacturing of the fullerene C₆₀ epoxide denoted by C₆₀O₃ of the present invention by the following manufacturing method.

### (1) Preparation of a fullerene epoxide mixture

In the manufacturing method of the present invention, a fullerene C₆₀ epoxide mixture is employed as the starting material. This fullerene C₆₀ epoxide mixture must contain the fullerene C₆₀ triepoxide being targeted. Such mixtures can be obtained, for example, by oxidizing fullerene C₆₀ with *m*-chloroperoxybenzoic acid (m-CPBA). The oxidation reaction with m-CPBA can be conducted under the following conditions, for example.

Molar ratio of fullerene C₆₀ and m-CPBA: 1:10 to 1:100, preferably 1:30 to 1:60.
Reaction temperature: 80 to 120°C

Reaction time: 1 to 60 min., preferably 10 to 30 min.

In addition to the above method employing a peroxybenzoic acid such as m-CPBA above, the mixture of fullerene C₆₀ epoxides can be prepared by methods employing an oxidizing agent in the form of organic peracids such as furanperoxide, dioxysilane compounds, ozone, P₄₅₀:cytochromeoxidase, and the like.

### (2) Separation of the fraction containing only triepoxides

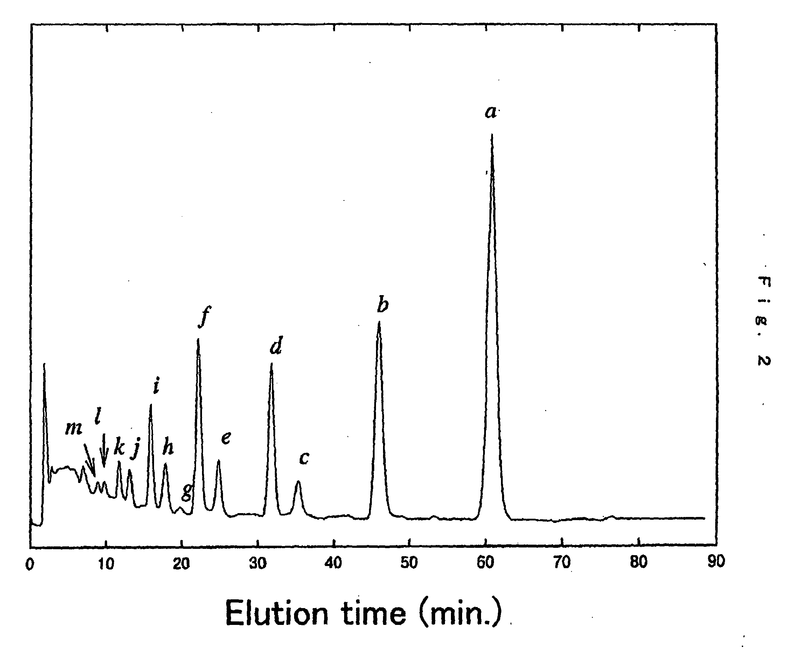

Generally, unreacted fullerene and low and high-order epoxides are present in fullerene C₆₀ epoxide mixtures. For example, the various fractions shown in Fig. 2 are present when a fullerene C₆₀ epoxide mixture is subjected to HPLC. The mass numbers of the individual fractions can be determined using, for example, LC-APCI-MS (liquid chromatography (LC) - atmospheric pressure chemical ionixation (APCI) - mass spectroscopy (MS): a form of high-performance liquid chromatography linked with an atmospheric chemical ionization mass analyzer).

In the method of the present invention, a fraction containing just fullerene triepoxides is separated with silica gel by LC-APCI-MS. Specifically, a mixture of fullerene C₆₀ epoxides is placed on a column packed with silica gel, a suitable eluant is used to sequentially separate out individual fragments, and a fragment containing only the targeted fullerene C₆₀ triepoxide is obtained.

The silica gel employed is not specifically limited. However, an alkane group-bonded silica gel is preferred. Examples of alkane group-bonded silica gels are C18 (octadodecyl) group-bonded silica gel and C30 group-bonded silica gel.

The eluant may be suitably selected based on the silica gel employed. For example, a mixed solvent of toluene and acetonitrile, a mixed solvent of toluene and methanol, or a mixed solvent of o-dichlorobenzene and methanol may be employed as a hydrophobic mobile phase.

Since the three fullerene C₆₀ epoxides having C₁, C₂, or Cₛ symmetry of the present invention have similar structures, as shown in Fig. 1, separation into three components with the above-described silica gel is essentially impossible. Specifically, when separation by silica gel was conducted with a reverse-phase Develosil C30 RPFULLERENE column, a fraction was containing just the fullerene C₆₀ triepoxide with peak e or f in Fig. 2 was obtained. When the peak refinement technique employed in electronic spectra was employed (L. Huber, "Application of diode array detection in HPLC," Hewlett-Packard Primer, Publication No. 12-5953-233, 1989), peak f was not pure, but contained multiple components.

Accordingly, the fraction containing just fullerene C₆₀ triepoxides obtained in the above step is further separated by a substance having π-π interaction with peak f in the present invention. Examples of substances having π-π interaction are pyrene-modified silica gels. Pyrene-modified silica gels are sold by Nacalai Tesque (Ltd.) as Cosmosil Bucky Prep columns, for example. An example of a substance having π-π interaction other than pyrene-modified silica gel is phenyl group-bonded silica gel.

Specifically, the fraction containing only fullerene C₆₀ triepoxides is placed on a column packed with pyrene-modified silica gel, a suitable eluant is employed to sequentially separate out various fragments, yielding fractions f and e as shown in Fig. 2. The eluant may be suitably determined based on the type of pyrene-modified silica gel employed. For example, a hydrophobic mobile phase in the form of toluene, acetonitrile, a mixed solvent of toluene and acetonitrile, a mixed solvent of toluene and methanol, or a mixed solvent of o-dichlorobenzene and methanol may be employed.

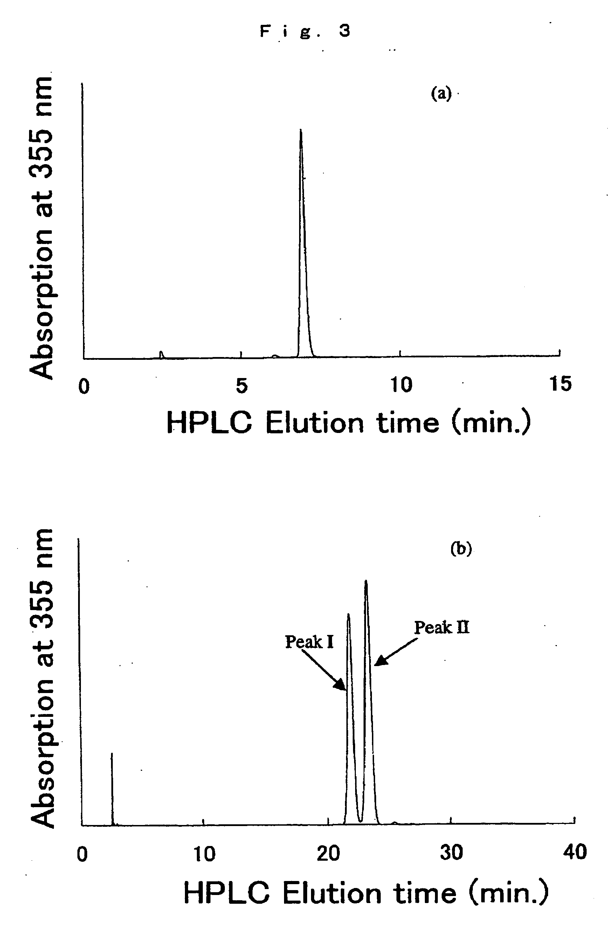

The top portion of Fig. 3 shows a chromatogram obtained when peak f was separated out with toluene/methanol (60:40) on the same Develosil C30 RPFULLERENE column as employed in silica gel separation. With silica gel, peak f yields a single fraction. By contrast, peak f is separated into peaks I and II in the chromatogram obtained by separation with toluene/methanol (80:20) on a Cosmosil Bucky Prep column, as shown in the bottom portion of Fig. 3.

The fraction in peak e obtained above and the fractions in peaks I and II were identified by the following method.

First, since the compounds contained in the fraction of peak e and the compounds contained in the fraction of peak II exhibited an isotope ratio matching the expected value for carbon isotopes and a mass spectrum having a main peak with a mass number of 768 (M⁻) in LC-APCI-MS measurement, and an absorption spectrum similar to that of C₆₀ in spectrochemical analysis in the ultraviolet and visible regions, they were identified as C₆₀O₃ isotopes.

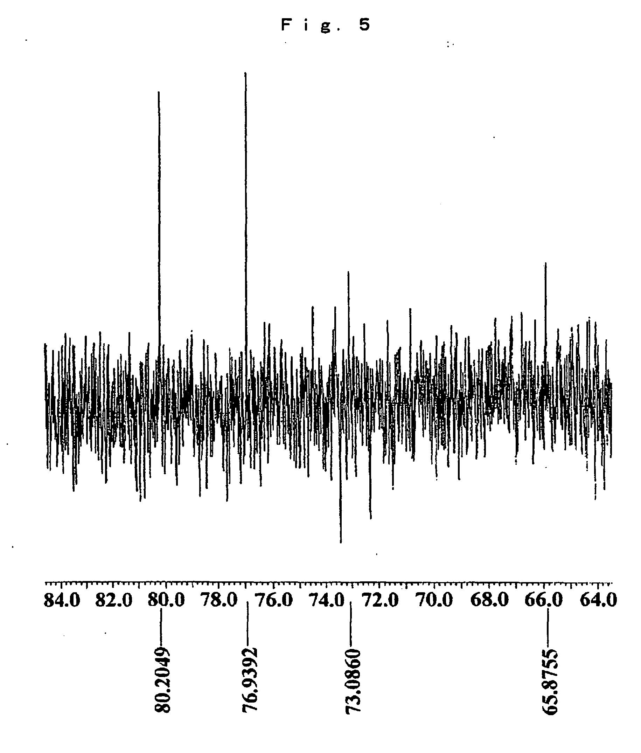

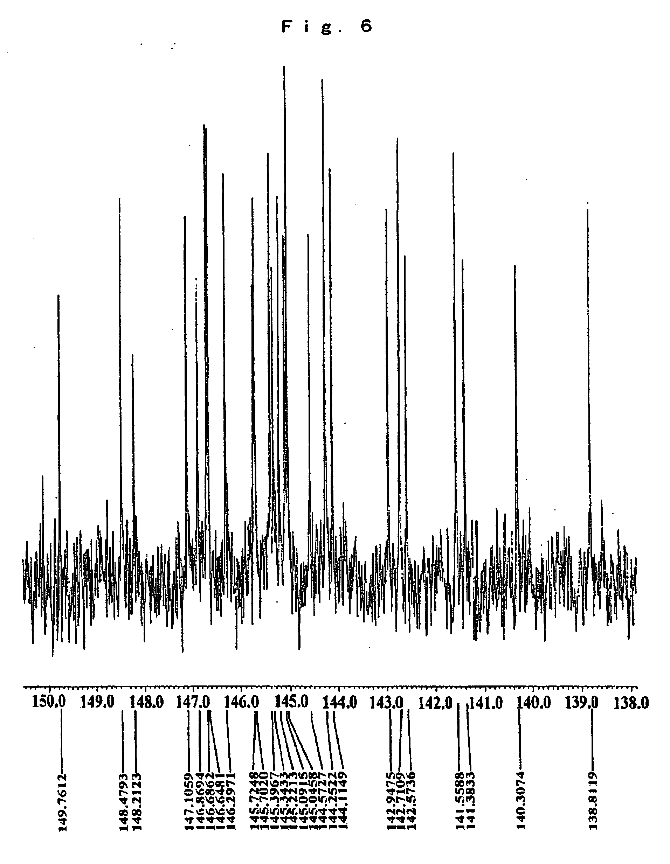

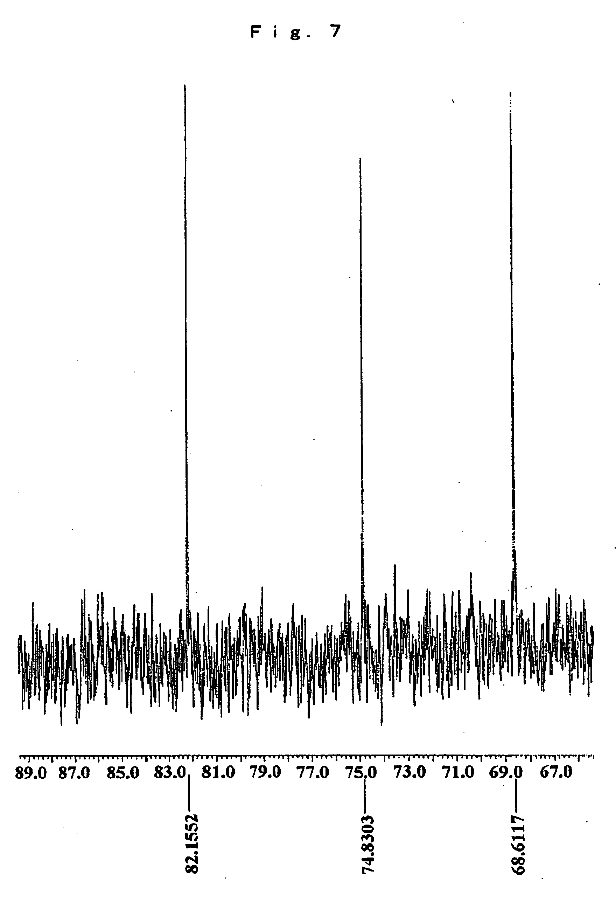

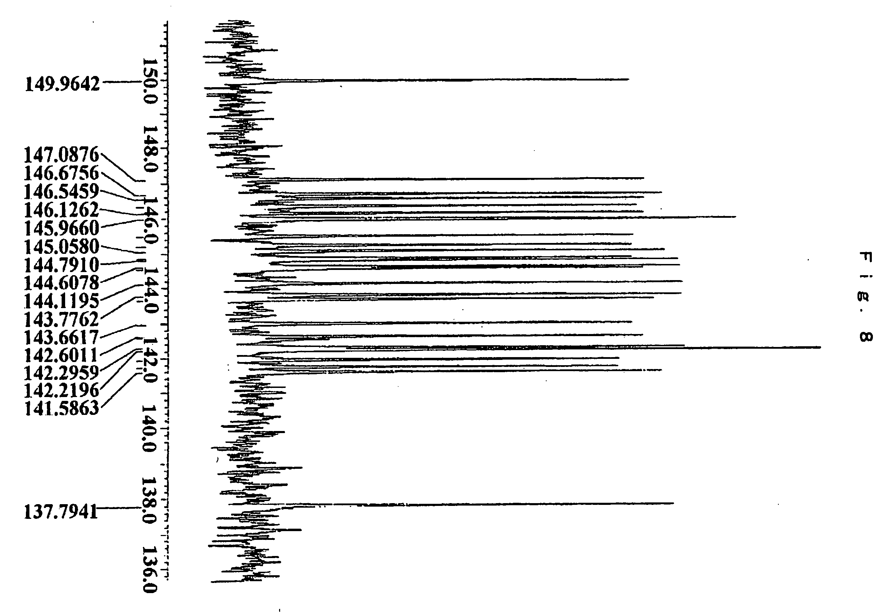

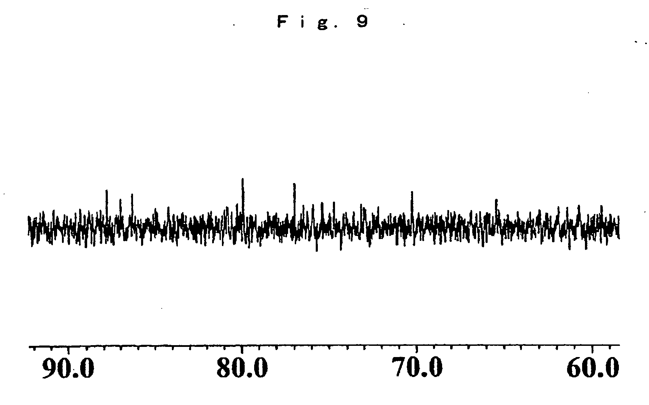

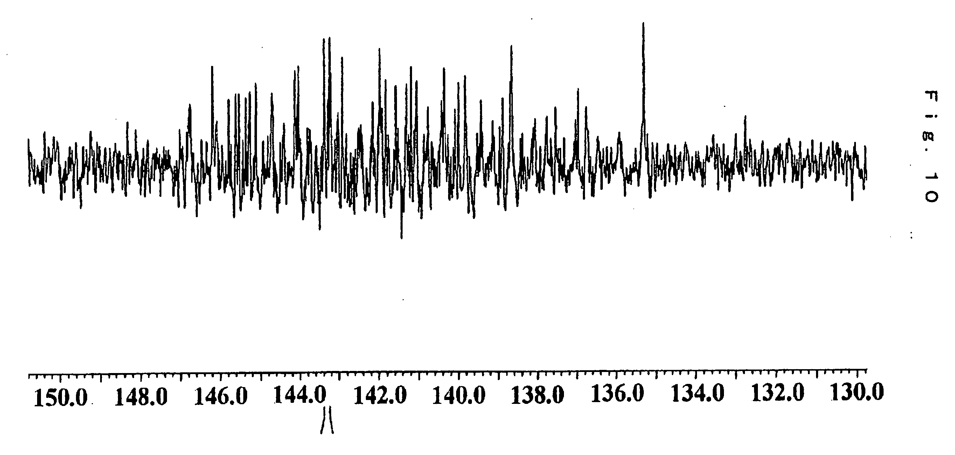

Next, ¹³C-NMR spectra were measured. The measurement method is described in detail in the embodiments. As a result, the fraction of peak e was determined to be three fullerene C₆₀ epoxides having C₁ symmetry (Figs. 9 and 10), the fraction of peak II was determined to be three fullerene C₆₀ epoxides having C₂ symmetry (Figs. 7 and 8), and the fraction of peak I was determined to be three fullerene C₆₀ epoxides having Cₛ symmetry (Figs. 5 and 6).

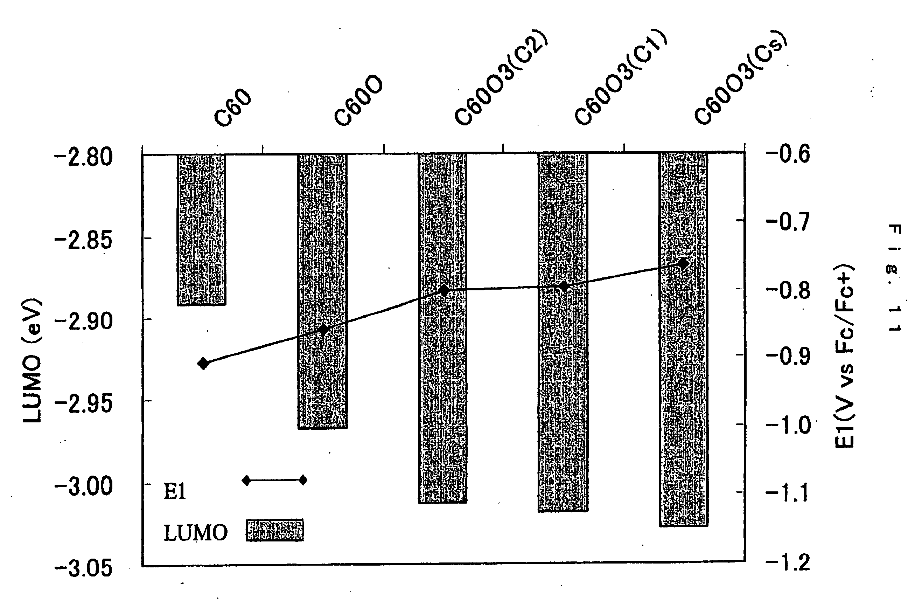

Measurement of the first reduction potentials (E1) of each revealed that peak I was -0.762 (V vs Fc/Fc⁺), while peak II and peak e had lower values of -0.796 and - 0.802, respectively (Fig. 11). As shown in Fig. 11, since the correlation between the lowest unoccupied molecular orbit (LUMO) levels (-3.018, -3.013, and -3.029 (eV)) calculated by semiempirical molecular orbit computation for the isomers of C₆₀O₃ having C₁, C₂, and Cₛ symmetry matched, peak I was identified as fullerene C₆₀ triepoxide having Cₛ symmetry, peak II was found to be fullerene C₆₀ triepoxide having C₂ symmetry, and peak e was found to be fullerene C₆₀ triepoxide having C₁ symmetry.

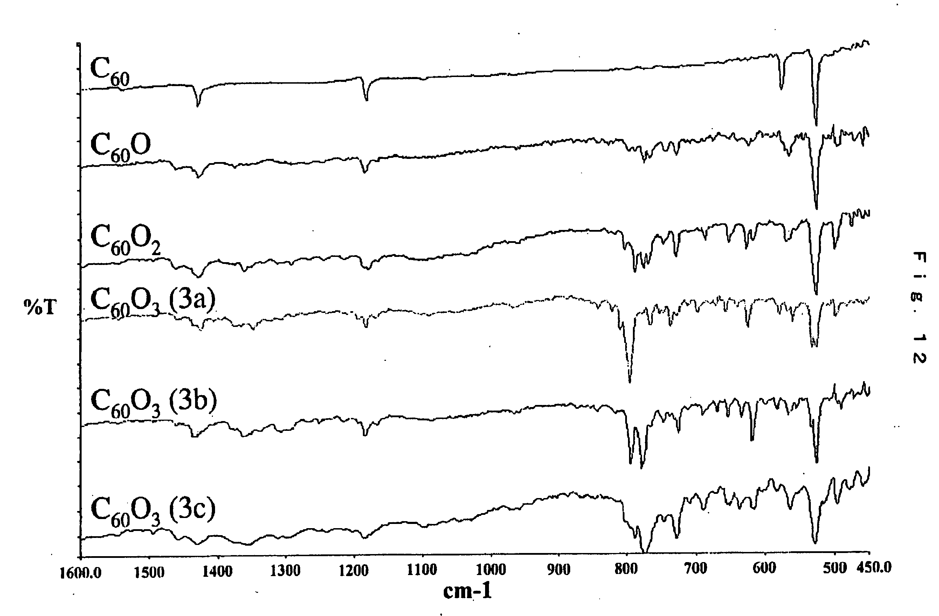

Further, oscillation peaks due to epoxy groups were observed in the vicinity of 800 cm⁻¹ in the FT-IR spectra (Fig. 12) of all of the components contained in the above fractions, indicating that these components had epoxy groups.

The various fullerene C₆₀ epoxides having C₁, C₂, and Cₛ symmetry obtained by the above-described manufacturing method were all racemics. Each racemic yields an enantiomer (mirror-image isomer) by optical resolution.

Since optical isomers have identical properties except for their optical activity, they cannot be resolved and refined by the usual methods. Generally, resolution is possible by high-performance liquid chromatography (HPLC) equipped with an optical resolution column. Known fillers employed in optical resolution columns include: 1) those having opposite-hand right-winding and left winding helical structures, such as polyamino acids, and 2) polymer adsorption agents in which derivatives of polyglutamic acid are fixed by covalent bonds to polystyrene beads. In the case of polyamino acids, the space formed between the polymer side chains forms opposing hands, with strong affinity for one of the optical isomers, resolving D and L forms. In the case of polymer adsorption agents, varying the type of amino acid derivative permits the preparation of adsorption agents capable of separating various optical isomers.

The fullerene C₆₀ triepoxides having C₁, C₂, and Cₛ symmetry of the present invention, particularly fullerene C₆₀ triepoxides having Cₛ symmetry, exhibit a greater electrophilic property than fullerene and are useful as electrical conductors, such as the electron receptors in photoelectric batteries and photoelectrical conductive materials. Further, since the fullerene C₆₀ triepoxides having C₁, C₂, or Cₛ symmetry of the present invention are epoxides and exhibit a pronounced electrophilic property, they are also useful as strong oxidizing agents.

In separation by the above-described substances having π-π interaction, not only is it possible to separate fullerene C₆₀ triepoxides, but it is also possible to separate other fullerenes such as C₇₀, C₇₂, C₇₆, and C₈₂ epoxides. An example of a substances having π-π interaction employed in such separation is pyrene-modified silica gel marketed by Nacalai Tesque (Ltd.) as Cosmosil Bucky Prep column. In addition to pyrene-modified silica gel, phenyl group-bonded silica gel and the like can be employed as a substance having π-π interaction.

### Embodiments

The present invention is described in greater detail below through embodiments.

A toluene solution of 1 x 10⁻³ mol/dm⁻¹ fullerene C₆₀ (purity of 99.98 percent or greater, Matsubo) and 1 x 10⁻¹ mol/dm⁻¹ m-chloroperoxybenzoic acid (Tokyo Kasei) was heated for 30 min while being refluxed in the dark. The reaction product obtained was washed with methanol, and the unreacted m-chloroperoxybenzoic acid was removed. Next, flash gel column chromatography employing silica gel (FC40, Wako Junyaku) was used to separate the mixture of fullerene epoxides from the unreacted C₆₀.

The mixture of fullerene epoxides obtained was subjected to high-performance liquid chromatography with a reverse-phase Develosil C30 RPFULLERENE column. A detection wavelength of 335 nm and toluene:acetonitrile (70:30) were employed in elution. The chromatogram obtained is shown in Fig. 2. Peaks e and f in Fig. 2 were determined to be C₆₀O₃ from the mass number employing liquid chromatograph (LC)-atmospheric pressure chemical ionixation (APCI) - mass spectroscopy (MS) (LC-APCI-MS).

Next, the fraction corresponding to peak f in Fig. 2 was eluted with toluene/methanol (80:20) on a Cosmosil Bucky Prep column. The chromatogram obtained is shown in Fig. 3. The fraction corresponding to peak f in Fig. 2 was separated into the two peaks I and II shown in Fig. 3(b). The fractions corresponding to peaks I and II both exhibited APCI mass signals at m/z 768 (M⁻). Both were also found to be pure when tested by peak purity testing using an electron spectrum (L. Huber, "Application of diode array detection in HPLC," Hewlett-Packard Primer, Publication No. 12-5953-233, 1989).

The molar yields (based on the amount of fullerene consumed) of each of the fractions of peak e, peak I, and peak II were 0.25 percent, 0.25 percent, and 0.35 percent, respectively.

Each of the fractions that was separated was dried, yielding brown powders. Toluene/acetonitrile solutions of each of the isomers isolated were dried and solidified by evaporation, washed several times with hexane to remove oily impurities contained in the extended solutions, and thoroughly dried at less than or equal to 40°C in a vacuum dryer, yielding black powders.

Each of these powders had low solubility in toluene and could not be measured by ¹³C-NMR by the usual methods. Accordingly, in the present invention, 5 mg of the black powder obtained was dissolved in 0.4 cc of carbon disulfide. A 0.1 cc quantity of benzene-d6, 1 mg of chromium (III) acetyl acetonate, and tetramethylsilane (TMS) were added, and the mixture was charged to an NMR tube and employed as a measurement sample. The measurement was conducted a total of 40,000 times using a Nippon Bunko JEOL GSX500 (125.7 MHz) indoors with a pulse width of 4.3 microseconds, an observation time band of 1.04 s, and a delay time of 0.5 s. The results are given in Figs. 5 to 10.

In the results of Fig. 5, the 84.0 to 64.0 region is the sp³ carbon region. The 150.0 to 138.0 region of Fig. 6 is the sp² carbon region. A total of four peaks in the form of two strong, clear peaks and two weak peaks observed in the sp³ carbon region indicated that this isomer was fullerene C₆₀ triepoxide having Cₛ symmetry.

The 89.0 to 67.0 region of Fig. 7 is the sp³ carbon region. The 150.0 to 136.0 region in Fig. 8 is the sp² carbon region. The clear three peaks observed in the sp³ carbon region indicated that this isomer was fullerene C₆₀ triepoxide having C₂ symmetry.

The 90.0 to 60.0 region in Fig. 9 is the sp³ carbon region and the 150.0 to 130.0 region in Fig. 10 is the sp² carbon region. Seven peaks were observed in the sp³ carbon region, one of which was thought to be an impurity. As a result, the observation of six peaks indicated that this isomer was fullerene C₆₀ triepoxide having C₁ symmetry.

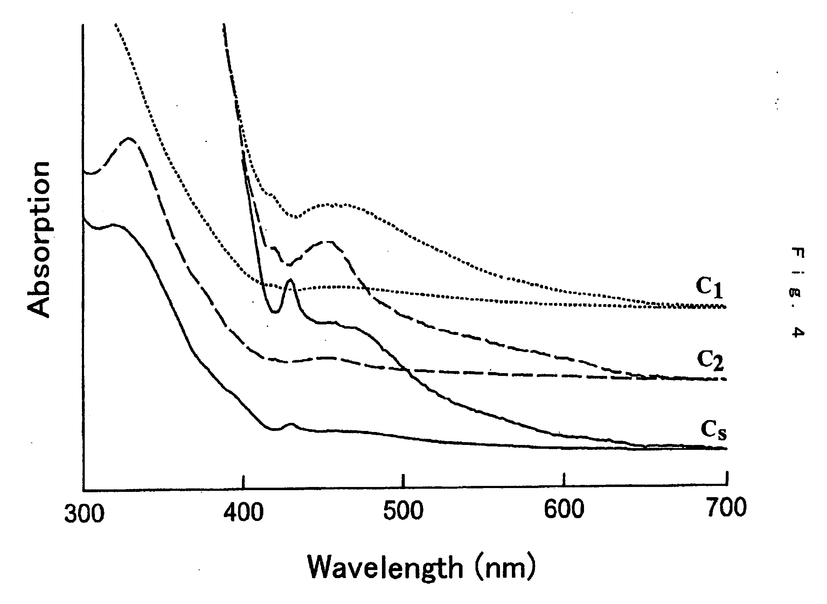

As shown in Fig. 4, the visible absorption spectrum of the toluene solution varied greatly between the fractions of peaks I and II. In particular, the powder obtained from the fraction of peak I had a sharp peak at 435 nm.

Further, FT-IR spectral measurements were made by the KBr pellet method on the above-described powder solid with a Spectrum One made by Perkins-Elmer Co. The results are given in Fig. 12.

In the FT-IR spectrum shown in Fig. 12, each of the powders obtained from the fractions of peaks I, II, and e exhibited absorption corresponding to C-O expansion oscillation in the vicinity of 800 cm⁻¹. These results identified the powder obtained from the fraction of peak I as fullerene C₆₀ triepoxide having Cₛ symmetry.

Further, the first reduction potential (E1) of each fraction was measured by the following method. The reduction potential (E1) was measured for samples in which 0.1 M (t-butyl)₄N⁺PF₆⁻ was added as a supporting electrolytic salt to a o-dichlorobenzene solution (1 x 10⁻³ M) of each of the fullerene C₆₀ triepoxides by differential pulse voltammetry (DPV). The scanning speed was 100 mV/s, platinum was employed in the working electrode, silver-silver chloride was employed in the reference electrode, and platinum wire was employed in the auxiliary electrode.

As a result, peak I was -0.762 (V vs Fc/Fc⁺), while peaks II and e were a low - 0.796 and -0.802, respectively. Since this correlated with the lowest unoccupied molecular orbit (LUMO) levels (shown in Fig. 11: -3.018, -3.013, and -3.029 (eV), respectively) calculated by semiempirical molecular orbit computation for each of the isomers of C₆₀O₃ having C₁, C₂, and Cₛ symmetry, peak I was found to be fullerene C₆₀ triepoxide having Cₛ symmetry, peak II was found to be fullerene C₆₀ triepoxide having C₂ symmetry, and peak e was found to be fullerene C₆₀ triepoxide having C₁ symmetry.

The calculation of the lowest unoccupied molecular orbit (LUMO) level obtained by semiempirical molecular orbit computation was performed by the PM3 method with WinMOPAC Version 3.0 (Fujitsu).

### Methods of optically resolving the various isomers

The concentration of the toluene solution containing the various isomers was about 1.1 mmol/L. A chemical bond Chiralpack AD column was prepared, and a UV and CD dual detector (JASCO CD-1595) and a recycle valve unit (HV-1592-01) were equipped. The sample was injected into the chromatography system with a Rheodyne model 7125 injector. A hexane-CHCl₃ (80:20) mixture was employed as eluant (flow rate 1.0 mL/min). The UV spectrum of the toluene solution was measured with a Hitachi U-2000 spectrophotometer.

The isomer (3c) was optically resolved by the above-described method.

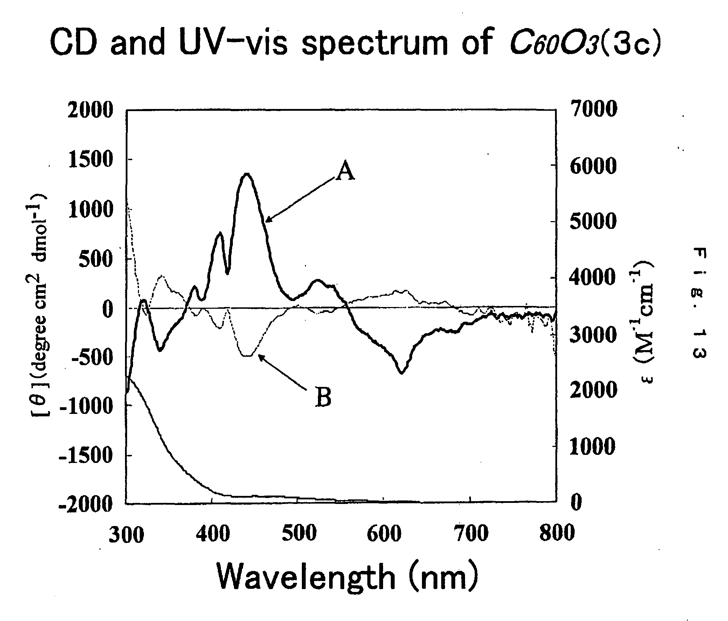

The CD spectra of the various optical activators of C₆₀O₃ isomer (3c) were measured using 0.2 cm quartz cells in hexane-CHCl₃ and toluene at room temperature with a JASCO J-720L. The results are given in Fig. 13. In Fig. 13, the enantiomer (3c) denoted by A is a positive compound, while the enantiomer (3c) denoted by B is a negative compound.

An attempt was also made to optically resolve isomers (3a) and (3b) by the method employing the above-described AD column, but no optical activator could be obtained..

### Industrial Applicability

The present invention provides fullerene C₆₀ epoxides having C₁ symmetry.

The present invention also provides a method of manufacturing these fullerene C₆₀ epoxides.

The present invention further provides a method of separating fullerene epoxides.

## Claims

1. A fullerene epoxide denoted as C₆₀O₃ having C₁ symmetry, **characterized by** the presence of two epoxide groups in a single six-membered ring, and by the presence of one epoxide group in a six-membered ring adjacent to the six-membered ring in which said two epoxide groups are present or by the presence of one epoxide group in a six-membered ring one six-membered ring removed from the six-membered ring in which said two epoxide groups are present.

2. The fullerene epoxide according to claim 1, wherein said epoxide is in the form of a racemic or an enantiomer.

3. A method of manufacturing fullerene epoxide comprising:
i) separating a fraction containing only fullerene triepoxides from a mixture of fullerene epoxides using silica gel, and
ii) separating the fraction containing only fullerene triepoxides thus obtained using a substance having π-π interactions selected from pyrene-modified silica gel and phenyl group-bonded silica gel to obtain fullerene epoxide according to any of claims 1 to 2.

4. The method of manufacturing according to claim 3, wherein said silica gel in step i) is alkane group-bonded silica gel.

## Patentansprüche

1. Als C₆₀O₃ bezeichnetes Fullerenepoxid mit einer C₁-Symmetrie, wobei dieses durch die Anwesenheit von zwei Epoxidgruppen in einem einzelnen sechsgliedrigen Ring und durch die Anwesenheit einer Epoxidgruppe in einem sechsgliedrigen Ring, welcher zu dem sechsgliedrigen Ring, in dem die beiden Epoxidgruppen anwesend sind, benachbart ist, oder durch die Anwesenheit einer Epoxidgruppe in einem sechsgliedrigen Ring, welcher einen sechsgliedrigen Ring von dem sechsgliedrigen Ring entfernt ist, in dem die beiden Epoxidgruppen anwesend sind, **gekennzeichnet** ist.

2. Fullerenepoxid nach Anspruch 1, wobei das Epoxid in der Form eines Racemats oder eines Enantiomers vorliegt.

3. Verfahren zum Herstellen von Fullerenepoxid umfassend:
i) Abtrennen einer Fraktion, welche lediglich Fullerentriepoxide enthält, aus einer Mischung von Fullerenepoxiden unter Verwendung von Silicagel und
ii) Trennen der Fraktion, welche lediglich so erhaltene Fullerentriepoxide enthält, unter Verwendung einer Verbindung mit π-π-Wechselwirkungen ausgewählt aus Pyren modifiziertem Silicagel und Silicagel mit Phenylgruppenbindungen, um Fullerenepoxid nach Anspruch 1 oder 2 zu erhalten.

4. Verfahren zum Herstellen nach Anspruch 3, wobei das Silicagel in dem Schritt i) Silicagel mit gebundener Alkangruppen ist.

## Revendications

1. Epoxyde de fullerène indiqué comme C₆₀O₃ ayant une symétrie C₁, **caractérisé par** la présence de deux groupes époxydes dans un cycle unique à six membres, et par la présence d'un groupe époxyde dans un cycle de six membres adjacent au cycle de six membres dans lequel lesdits deux groupes époxydes sont présents ou bien par la présence d'un groupe époxyde dans un cycle de six membres, un cycle de six membres retiré du cycle de six membres dans lequel lesdits deux groupes époxydes sont présents.

2. Epoxyde de fullerène selon la revendication 1, où ledit époxyde se présente sous la forme d'un racémique ou d'un énantiomère.

3. Procédé de fabrication d'un époxyde de fullerène comprenant:
i) séparer une fraction contenant seulement des triépoxydes de fullerène d'un mélange d'époxydes de fullerène en utilisant du gel de silice, et
ii) séparer la fraction contenant seulement les triépoxydes de fullerène ainsi obtenus en utilisant une substance ayant des interactions π-π sélectionnées dans le gel de silice pyrène-modifié et le gel de silice lié au groupe phényle pour obtenir un époxyde de fullerène selon l'une quelconque des revendications 1 à 2.

4. Procédé de fabrication selon la revendication 3, où ledit gel de silice à l'étape i) est un gel de silice lié au groupe d'alcane.
